Europäisches Patentamt

European Patent Office

Office européen des brevets

(19)

(11) Numéro de publication: **0 300 286**
**A2**

## (12) DEMANDE DE BREVET EUROPEEN

(21) Numéro de dépôt: 88110911.0

(22) Date de dépôt: 08.07.88

(51) Int. Cl.⁴: **B32B 27/18**

(30) Priorité: 22.07.87 CH 2781/87

(43) Date de publication de la demande:
25.01.89 Bulletin 89/04

(84) Etats contractants désignés:
**BE CH DE ES FR GB IT LI NL**

(71) Demandeur: **FIRMENICH SA**
**1, route des Jeunes Case Postale 239**
**CH-1211 Genève 8(CH)**

(72) Inventeur: **Holzner, Günter**
**15, chemin des Palettes**
**CH-1212 Grand-Lancy(CH)**

(74) Mandataire: **Salvadori, Giuseppe, Dr.**
**c/o Firmenich S.A. Case Postale 239**
**CH-1211 Genève 8(CH)**

(54) **Feuille sandwich en couches multiples et emballage faisant usage de ladite feuille.**

(57) Feuille sandwich laminée en couches multiples comportant deux couches au moins superposées et accolées par soudure ou collage et dont l'une d'entre elles est constituée par un matériau imperméable aux agents volatils actifs d'une composition parfumante ou désodorisante, d'un arôme ou d'une substance insecticide ou bactéricide et l'autre se compose d'un film réalisé à partir d'un polymère ou d'un copolymère à base de polyoléfine ou d'un copolymère de polyamide et polyéther, lequel film contient, en phase dispersée et homogène, une base constituée par une composition parfumante ou désodorisante, un arôme ou une substance insecticide ou bactéricide et procédé pour sa fabrication.

Emballage fabriqué à l'aide de ladite feuille.

EP 0 300 286 A2

# Feuille sandwich en couches multiples et emballage faisant usage de ladite feuille

La présente invention a trait au domaine de l'emballage et du conditionnement. Elle a en particulier pour objet un emballage industriel pour produits ou matériaux pouvant dégager des vapeurs volatiles ou auxquels, tout au moins, il est souhaité conférer une odeur pendant la période qui précède leur consommation ou activation et pendant laquelle le produit se trouve sous emballage fermé. Il s'agit par exemple de produits cosmétiques, de produits parfumés variés, de désodorisants d'air ambiant, de détergents ou adoucisseurs textiles, de papier, de textiles, d'insecticides, d'arômes, d'aliments ou du tabac.

Comme il apparaîtra de manière plus détaillée dans la suite de cette description, l'emballage de l'invention constitue un support, unique ou complémentaire, apte à la diffusion de vapeurs volatiles ou assainissantes et de ce fait il est destiné en général à améliorer la perception auprès du consommateur des produits ou matériaux emballés.

Il est incontesté que les propriétés odorantes d'un produit exercent souvent un effet déterminant sur le consommateur. Dans bien des cas et pour une certaine catégorie de produits, elles définissent de façon souvent inconsciente la propension à l'achat.

Les fabricants se sont attelés à augmenter ce signal en essayant de conférer ou renforcer les caractères odorants des produits offerts à la consommation. Toutefois, ceci se heurte à des limites pratiques évidentes. Il est en effet difficile d'augmenter l'intensité odorante d'un produit au-delà de certaines limites sans que ceci ait une influence négative sur l'harmonie olfactive qu'on souhaiterait atteindre. En d'autres termes, l'odeur d'un produit doit être perceptible et plaisante et par cela même conférer au produit un caractère distinctif sans pour autant s'imposer.

Il est en outre connu que le consommateur attache beaucoup d'importance à la première impression qu'il reçoit d'un produit. Le parfum qui se dégage d'un produit fraîchement déballé peut exercer sur lui une impression déterminante quant à l'acceptation du produit même. On connaît différentes solutions qui consistent à parfumer le matériel d'emballage et plusieurs procédés ont été suggérés à cet effet.

Il existe dans le commerce des matériaux d'emballage parfumés. Il s'agit généralement de papier, carton ou plastique. Ce dernier matériau a fait l'objet d'une attention particulière. Grâce à la mise en application de la technique dite du "master-batch", on prépare des concentrés d'agent parfumant dans des matrices constituées par la même résine polymérique, généralement polyoléfinique, que celle qui est utilisée pour la préparation du produit final. On peut mentionner par exemple que des produits à matrice polyoléfinique pour procédé "master-batch" caractérisés par un contenu élevé en substances volatiles parfumantes sont récemment apparus sur le marché. Il s'agit de résines à base de polyéthylène à basse densité (LDPE), de polypropylène et de copolymères d'éthylène-vinyl acétate [voir par exemple : Polyiff (marque enregistrée) ; origine : Int. Flavors & Fragrances, Inc.].

Ces concentrés polyoléfiniques sont préparés généralement par incorporation du parfum dans des mélangeurs appropriés à des températures variables en fonction de la base polymérique. Le polyéthylène, par exemple, est traité à une température comprise entre 80 et 180 °C, ce qui permet d'obtenir une masse visqueuse à laquelle on additionne le parfum désiré avant le refroidissement et la granulation. Un tel procédé se heurte à une difficulté pratique majeure, l'incorporation du parfum à une température élevée entraîne en effet des pertes et des modifications olfactives de certains de ces composants, ce qui limite le choix de bases parfumantes appropriées.

Le parfumage de feuilles plastiques d'autre part rencontre également des problèmes majeurs. En effet, la fabrication de feuilles s'effectue essentiellement par l'une des méthodes suivantes :

a. extrusion par couchage

b. extrusion à filière plate

c. extrusion par soufflage.

Dans chacune de ces méthodes, le polymère est soumis à un traitement thermique plus ou moins prolongé suivi d'une compression au travers d'une buse de géométrie appropriée. Lorsque cette compression est accompagnée par une injection d'air ou de gaz inerte et que la buse possède une forme annulaire, comme c'est le cas dans l'extrusion par soufflage, on obtient un produit sous forme de cylindre dont les parois sont constituées par le matériau plastique désormais réduit en couche fine et d'épaisseur homogène. Par coupure longitudinale, on obtient ensuite des feuilles mono- ou multi-couches. Or le parfumage des feuilles au moyen d'un concentré de parfum sous forme de master-batch est mal adapté aux procédés que nous venons de décrire car, dans chacune des variantes mentionnées, le parfum subit des contraintes thermiques importantes, ce qui entraîne dans bien des cas une dénaturation. Ce phénomène est par ailleurs encore plus prononcé dans le procédé d'extrusion par soufflage où l'action combinée de la chaleur et du courant d'air chaud provoque une évaporation importante des constituants volatils d'une base assainissante à partir de la surface du film chaud qui est exposée directement à l'air ambiant.

La présente invention apporte une solution au problème exposé. La feuille polymérique qui résulte du procédé de l'invention possède en effet les caractéristiques assainissantes ou aromatisantes souhaitées et peut être employée en tant que matériel d'emballage plastique. Le procédé de l'invention ne requiert pas l'utilisation d'un équipement particulier et ce fait ne représente pas son moindre avantage.

L'invention a plus particulièrement pour objet une feuille sandwich laminée en couches multiples comportant deux couches au moins superposées et accolées par soudure ou collage et dont l'une d'entre elles est constituée par un matériau imperméable aux agents volatils actifs d'une composition parfumante ou désodorisante, d'un arôme ou d'une substance insecticide ou bactéricide et l'autre se compose d'un film réalisé à partir d'un polymère ou un copolymère à base de polyoléfine ou d'un copolymère de polyamide et polyéther d'une épaisseur de 10 à 300 miorons, lequel film contient, en phase dispersée et homogène, une base constituée par une composition parfumante ou désodorisante, un arôme ou une substance insecticide ou bactéricide dans une proportion de 0,1 à 50% en poids par rapport au poids de la base polymérique dans laquelle elle est incorporée.

A titre de résine polyoléfinique pour la préparation du film de support pour la base active, on peut utiliser du polyéthylène, du polypropylène, des copolymères d'éthylène et propylène ou des copolymères d'éthylène avec l'acétate de vinyle ou l'acrylate d'éthyle. A cet effet, le Surlyn (marque enregistrée de Du Pont de Nemours) convient parfaitement.

A titre de matériau imperméable aux agents volatils, on peut utiliser une résine polyamidique, un polyester, ou un film d'aluminium.

La feuille de l'invention peut être fabriquée à l'aide d'un procédé d'extrusion par soufflage, lequel procédé est caractérisé par l'extrusion simultanée, dans un courant d'air ou de gaz inerte, d'au moins deux matériaux polymériques dont l'un, à base d'une résine polyoléfinique ou réalisée à partir d'un copolymère de polyamide et polyéther est mélangé préalablement de façon homogène à une base active constituée par une composition parfumante ou désodorisante, un arôme ou une substance insecticide ou bactéricide et l'autre est constitué par un matériau polymérique imperméable aux agents volatils de ladite base active et en ce que l'extrusion donne lieu à un film polymérique à couches multiples accolées et disposées de sorte que la couche extérieure soit constituée par le matériau imperméable et la couche intérieure constituée par le matériau polymérique contenant la base active.

Comme indiqué plus haut, l'extrusion par soufflage a lieu sous l'effet d'un courant d'air. Lors de l'emploi de bases actives constituées par des compositions sensibles à l'oxydation, on peut effectuer aisément une telle extrusion par soufflage d'un gaz inerte. A cet effet, l'azote convient parfaitement.

Au terme de la présente invention, on entend par "base parfumante" toute substance ou mélange de substances parfumantes, tant à l'état isolé qu'en solution ou suspension, dans leurs diluants, dissolvants ou coingrédients habituels. Ces termes comprennent en particulier des solutions organiques généralement non-miscibles à l'eau dotées d'une tension de vapeur appréciable. De telles bases parfumantes peuvent être constituées par des composés appartenant à des classes chimiques distinctes et comprennent par exemple des esters, des éthers, des alcools, des aldéhydes, des cétones, des acétals, des nitriles, des hydrocarbures terpéniques, des composés hétérocycliques azotés ou soufrés ainsi que des huiles essentielles d'origine naturelle. Le choix particulier de la base parfumante dépend de l'effet odorant recherché, de la nature du produit que l'on désire mettre sous emballage et bien entendu du goût et de la préférence du parfumeur créateur.

Des exemples typiques de composés parfumants utiles sont donnés dans la littérature, et à cet effet, on peut citer S. Arctander, Perfume and Flavor Chemicals, Montclair, N.J. (USA), 1969.

Par "base désodorisante", on entend une substance capable de masquer l'odeur et d'inhiber la prolifération des bactéries, par exemple responsables de la dégradation de la sueur. Bon nombre de produits bactéricides et bactériostatiques sont connus et utilisés à cet effet. A titre d'exemple, on peut mentionner l'hexachlorophène, le dichlorophénol, le trichlorosalicylanilide (Anobial), le tribromosalicylanilide (TBS), le tétrachlorosalicylanilide (TCSA) et le trichlorocarbanilide (TCC).

A titre d'arôme, on peut employer l'une des compositions aromatisantes généralement utilisées pour conférer, améliorer ou modifier le goût et l'arôme d'aliments, boissons ou tabac. Le cas échéant, on peut bien entendu utiliser des substances aromatiques spécifiques isolées, telles les essences aromatisantes naturelles ou les composés synthétiques purs. Parmi les substances volatiles utiles qui peuvent être employées à cet effet, il convient de mentionner celles décrites dans les ouvrages spécialisés tel celui de S. Arctander (ouvrage cité plus haut) et le Fenaroli's Handbook of Flavor Ingredients, 2nd Edition, CRC Press, Cleveland, Ohio (1975).

A titre d'agent insecticide, on peut utiliser l'extrait de pyrèthre, le DDVP (origine : Ciba-Geigy, Bâle, Suisse) et le Vaporthrin (origine : Sumitomo, Osaka, Japon).

3

Comme il a été mentionné plus haut, la fabrication des feuilles polymériques suivant l'invention ne nécessite pas d'équipement particulier ; il suffit pour cela d'un appareillage normal pour l'extrusion par soufflage et la description détaillée d'une telle machine est ici superflue (voir les ouvrages spécialisés ; par exemple "Kunstoffverarbeitung" de Schwarz et al., Vogel-Verlag, 2ème Edition (1981), Würzburg (RFA).

Le mélange préalable de la résine polyoléfinique servant de support à la base active, parfumante, bactéricide ou autre, peut également se faire dans un appareillage simple usuel. La base active peut être prémélangée sous forme concentrée de façon à constituer un master-batch qui est ajouté à la masse de la résine polymérique soumise à extrusion. En effectuant l'opération à l'aide de deux, voire trois différentes sortes de résine polymérique, on peut opérer de sorte que le film résultant soit constitué par deux, le cas échéant trois couches accolées et disposées de façon à ce que la couche extérieure soit constituée par un matériau imperméable et la couche intérieure, le cas échéant la couche intermédiaire, soit constituée par le matériau polymérique contenant la base active.

Il en résulte que sous l'effet de protection exercé par la couche extérieure imperméable, les constituants volatils de la base active seront retenus sans évaporation notable dans la masse de la couche intérieure et ce malgré l'action d'entraînement exercé par le courant d'air chaud. La feuille résultante sera donc caractérisée par un contenu uniforme de base active de composition homogène et la dispersion de celle-ci sera pratiquement supprimée pendant tout l'opération d'extrusion et façonnage. Utilisée dans la fabrication d'emballages fermés, généralement souples, par soudure ou collage, une telle feuille présente l'avantage éminent de permettre la conservation de la base active pendant la période de stockage et de garder celle-ci à l'abri des influences extérieures telles l'oxydation ou l'action exercée par l'humidité du milieu environnant. Protégés par la couche extérieure imperméable, les constituants volatils de la base active seront ainsi retenus à l'intérieur de l'emballage jusqu'à son ouverture où ils se disperseront dans l'atmosphère environnante et exerceront leur action parfumante, désodorisante, bactéricide ou autre.

Alternativement, on peut employer la feuille polymérique de l'invention pour réaliser des emballages dont la couche extérieure serait constituée par le matériau polymérique contenant la base active et la couche intérieure par le matériau polymérique imperméable. Il s'agit là d'une application particulière dans tous les cas où l'on désire obtenir un emballage pouvant dégager une odeur, par exemple, tout en garantissant une étanchéité vis-à-vis du produit contenu dans l'emballage. Des formes d'exécution de l'emballage de l'invention seront décrites, à titre d'exemple, en se référant aux dessins annexés dans lesquels :

la fig. 1 représente une coupe transversale d'une feuille sandwich à deux couches ;

la fig. 2 représente une coupe transversale d'une feuille sandwich à deux couches accolées par collage ;

la fig. 3 représente une coupe transversale d'un emballage réalisé à l'aide d'une feuille sandwich à deux couches selon l'invention et contenant un produit parfumé sous forme granulaire ;

la fig. 4 représente une coupe transversale d'un emballage dont les parois sont constituées par une feuille sandwich à deux couches selon l'invention et contenant des mouchoirs en papier ;

la fig. 5 représente une coupe transversale d'un emballage dont les parois sont constituées par une feuille sandwich à deux couches selon l'invention et renfermant un produit sous forme granulaire ;

la fig. 6 représente schématiquement un appareillage pour extrusion par soufflage.

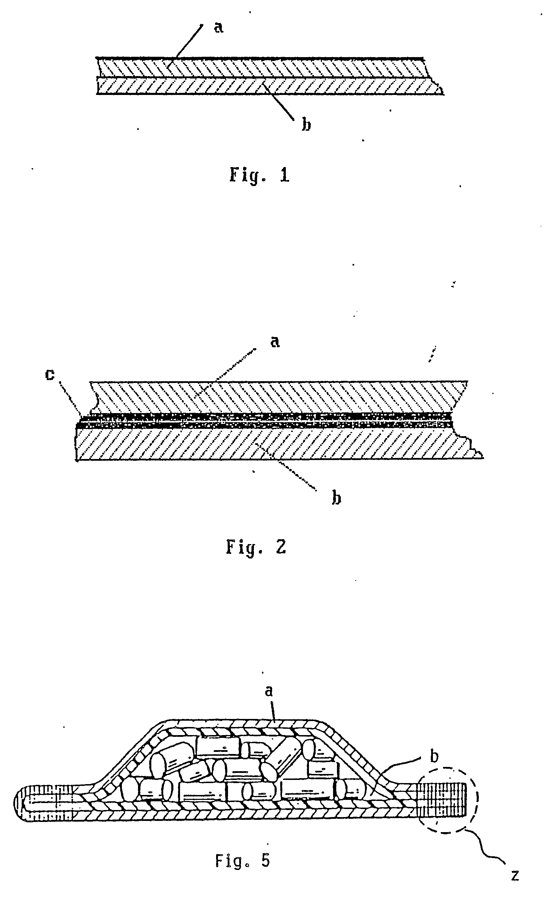

La feuille polymérique sandwich représentée à la fig. 1 se compose d'une couche (a) imperméable aux agents volatils d'une base active, laquelle couche est accolée à une couche (b) réalisée à partir d'un polymère à base de polyoléfine et contenant la base active. La fig. 2 représente une forme d'exécution particulière de la feuille selon l'invention. La couche imperméable (a) est accolée par collage à la couche perméable (b) à l'aide d'une substance adhésive (c).

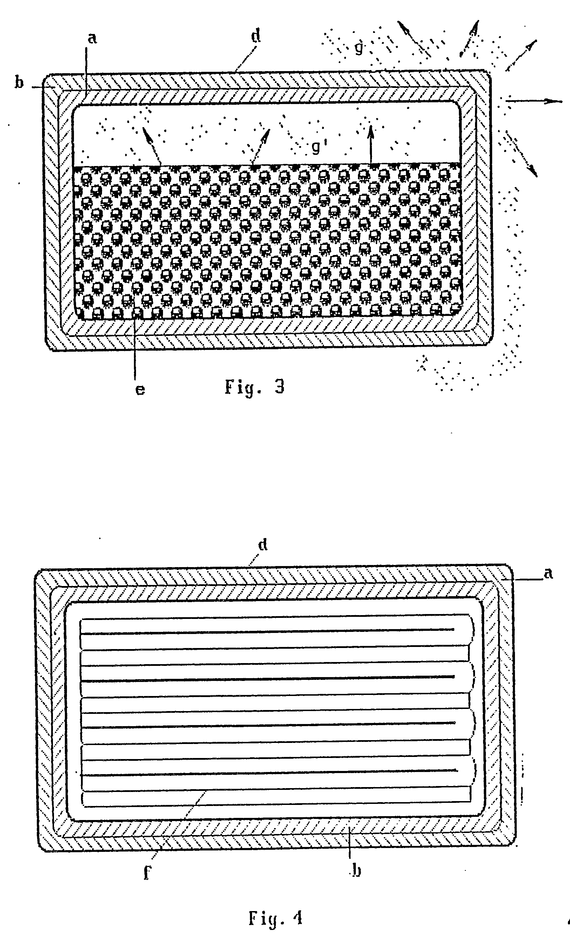

La fig. 3 représente un emballage (d) réalisé à l'aide de la feuille selon l'invention. L'emballage comporte des parois constituées par une feuille à deux couches et dont la couche intérieure (a) est imperméable aux vapeurs volatiles (g′) se dégageant du produit granulaire parfumé (e) contenu dans l'emballage, tandis que la couche extérieure (d) est réalisée à partir d'un polymère à base de polyoléfine et sert de support à une base active pouvant dégager des vapeurs assainissantes (g) dans l'atmosphère environnante.

La fig. 4 représente un emballage (d) réalisé à l'aide de la feuille selon l'invention et contenant des mouchoirs ou serviettes en papier (f). L'emballage comporte des parois constituées par une feuille à deux couches et dont la couche intérieure (b) sert de support à une base active pouvant dégager des vapeurs parfumantes et/ou bactéricides à l'intérieur de l'emballage, tandis que la couche extérieure (a) est imperméable auxdites vapeurs.

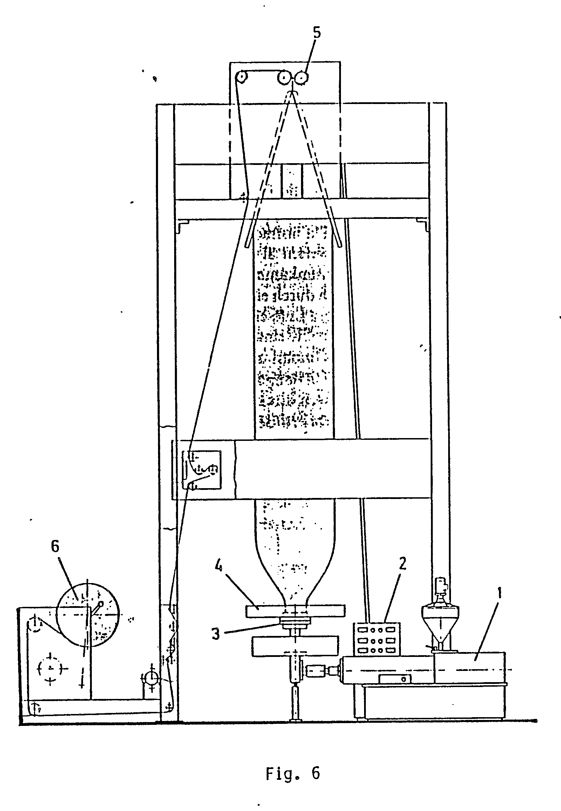

L'emballage représenté à la fig. 5 se compose d'un compartiment renfermant un produit sous forme granulaire, le compartiment étant fermé par la soudure (zone z) d'une feuille polymérique sandwich selon l'invention constituée par deux couches accolées : (a) imperméable aux vapeurs d'une base active, et (b) réalisée à partir d'un polymère polyoléfinique servant de support homogène à la base active.

La fig. 6 représente un schéma d'appareillage pour l'extrusion par soufflage servant à la préparation de la feuille polymérique sandwich selon l'invention. L'ensemble se compose d'une extrudeuse 1, d'un panneau de contrôle 2, d'une tête de soufflage 3, d'un anneau de refroidissement 4, d'un rouleau 5 pour retirer les feuilles et d'un système d'enroulement des feuilles 6.

Selon une forme d'exécution particulière, l'appareil d'extrusion par soufflage est composé par une série d'extrudeuses, chacune d'entre elles permettant l'extrusion d'un matériau polymérique distinct. Chacun des matériaux fondus est amené à une tête de soufflage séparée et disposée de manière concentrique, de sorte que, après soufflage, le rouleau extérieur soit constitué par le matériau polymérique imperméable et le rouleau intérieur par le matériau polymérique servant de support de diffusion pour la base parfumante, insecticide ou autre.

Lorsqu'on désire réaliser une feuille constituée par deux couches superposées et collées, on peut opérer à l'aide d'un système opérant avec trois extrudeuses dont deux servent à effectuer l'extrusion des résines polymériques choisies et la troisième à l'extrusion du produit de collage. A cet effet, on peut utiliser les matériaux polymériques usuels prévus dans l'art. L'Elvax (marque enregistrée de Du Pont de Nemours), résine à base d'un copolymère d'éthylène et vinylacétate, peut être employée à cet effet.

La fabrication de la feuille sandwich selon l'invention peut donc être aisément effectuée au moyen de la technique décrite plus haut d'extrusion par soufflage. Toutefois, les autres méthodes de fabrication des feuilles peuvent également être employées convenablement. C'est ainsi, que des feuilles à deux ou trois couches selon l'invention peuvent être fabriquées par extrusion par couchage. Cette méthode est particulièrement adaptée à la fabrication de feuilles dont la couche imperméable est constituée par une feuille d'aluminium par exemple. Selon les techniques conventionnelles, une telle feuille d'aluminium peut être ensuite recouverte, le cas échéant, par du papier et la feuille ainsi obtenue peut constituer un matériau adapté à la manufacture d'emballages standard pour aliments et boissons en particulier. En utilisant une feuille polymérique aromatisée à titre de couche intérieure, on peut ainsi conférer au contenu de l'emballage l'arôme souhaité.

L'invention est illustrée de manière plus détaillée par les exemples suivants dans lesquels les températures sont indiquées en degrés centigrades.

<center>Exemple 1</center>

A. Fabrication d'une feuille par extrusion par soufflage

Une feuille polymérique sandwich à base de polyéthylène et polyamide a été préparée au moyen d'un appareil d'extrusion par soufflage équipé de trois extrudeuses disposées en forme d'étoile dont les sorties sont réunies dans une tête de soufflage à trois buses (équipement pilote de la société Du Pont de Nemours, Genève).

Les trois extrudeuses ont été alimentées séparément par
    a. du polyamide (Durethan C38F ; Bayer AG),
    b. de l'adhésif E148 (Du Pont de Nemours), et
    c. du polyéthylène (Baylon 1555F14 ; Bayer AG).

Du polyéthylène granulé a été préalablement mélangé avec une base parfumante ou aromatisante à raison de 20 parties de base pour 80 parties de polyéthylène. Ce mélange, après brassage, a été laissé au repos pendant 24 heures à température ambiante de sorte que la base liquide soit totalement absorbée sur le support polymérique. Ce mélange a été ensuite ajouté à la masse de polyéthylène granulé avant extrusion dans une proportion de 2,5 g de mélange parfumé pour 97,5 g de polyéthylène.

Les résines polymériques a, b et c ont été extrudées de manière à constituer respectivement la couche extérieure, médiane et intérieure du film sortant. La température d'extrusion était de 210°. Le refroidissement du film sortant a été effectué à l'aide d'un courant d'air. Le film obtenu a été enroulé en boyau sans coupe et utilisé directement pour la fabrication d'emballage comme décrit ci-après dans les exemples donnés. Les trois couches avaient une épaisseur de 20, 10 et 25 microns respectivement pour la couche extérieures, intermédiaire et intérieure.

<center>5</center>

B. Fabrication d'une feuille par extrusion par couchage

Un appareil pilote de la société Du Pont de Nemours équipé de trois extrudeuses disposées en parallèle ayant chacune une buse plate pour l'extrusion d'un film mince de 50 cm de largeur a été employé pour une telle fabrication.

On a procédé d'abord à la fabrication d'une feuille à deux couches dont l'une était constituée par un film de 12 microns d'épaisseur d'aluminium et l'autre était constituée par du papier blanc de type 50 g/m², les deux couches étant collées à l'aide d'un adhésif standard. On a procédé séparément à la préparation d'un granulé concentré constitué par du copolymère de polyéthylène vinylacétate [Elvax (marque enregistrée) 150 ; Du Pont de Nemours] parfumé ou aromatisé avec une base appropriée et choisie en fonction du produit que l'on désire emballer (voir exemples ci-après). La proportion de la base par rapport au granulat polymérique était de 20 parties de base pour 80 parties de résine polymérique.

Le granulat parfumé ainsi obtenu a été mélangé avec la masse de Surlyn [(marque enregistrée) 1652 ; Du Pont de Nemours] avant extrusion à une concentration de 2,5% et la résine résultante a été extrudée par couchage sur la couche d'aluminium du film préalablement obtenu (voir plus haut) de manière à obtenir un film de 30 microns d'épaisseur. La température d'extrusion était de 235˚.

Le film résultant a été immédiatement refroidi sur un rouleau froid à la sortie de l'extrudeuse et enroulé.

## Exemple 2

## Parfumage d'un emballage pour mouchoirs en papier

Une feuille polymérique sandwich, préparée conformément au procédé décrit à l'Exemple 1A et constituée par une couche imperméable polyamidique de 20 microns et une couche de polyéthylène de 20 microns, cette dernière ayant été parfumée à raison de 0,5% en poids à l'aide d'une composition de type menthe (Menthe poivrée Naefco ; origine : Firmenich SA) ou cyprès (Cyprès 41.463 ; origine : Firmenich SA), a été utilisée pour emballer des mouchoirs en papier qualité Tela (marque enregistrée) standard. L'emballage a été réalisé par soudure des bords de la feuille au moyen d'une soudeuse à impulsion.

Dans un tel emballage, les mouchoirs acquirent après quelques jours déjà un parfum distinct caractéristique de la composition utilisée. Un tel parfum se dégage agréablement dans l'atmosphère environnante lors de l'ouverture de l'emballage.

## Exemple 3

## Parfumage d'un emballage pour fleurs artificielles en papier ou en soie

On a procédé à la préparation d'un emballage destiné à des fleurs artificielles en papier, respectivement en soie, à l'aide d'une feuille obtenue conformément à l'Exemple 1A et dont une couche a été parfumée avec une composition de type oeillet (Oeillet 660.895/B ; origine : Firmenich SA) à raison de 0,5% en poids.

Dans un tel emballage les fleurs avaient absorbé une partie des vapeurs volatiles de la composition parfumante et dégageaient une senteur agréable une fois sorties de leur emballage.

## Exemple 4

Parfumage d'un emballage pour linge et vêtements

On a procédé à la préparation d'un emballage destiné à contenir du linge comme indiqué à l'Exemple 1A en utilisant comme composition parfumante active une base cyprès (Cyprès 41.463 ; origine : Firmenich SA) et oeillet (Oeillet 660.895/B ; origine : Firmenich SA). Le linge ainsi emballé acquiert une odeur agréable, odeur qui se dégage à l'ouverture de l'emballage même après une période très prolongée (10 mois). En remplaçant la composition parfumante par un agent insecticide volatil [Vaporthrin (marque enregistrée) ; origine : Sumitomo, Osaka, Japon], ou un antimite usuel (naphtaline), on a obtenu un emballage dont l'action de protection vis-à-vis des agents extérieurs s'est révélée particulièrement efficace. Bien entendu, on peut également obtenir des feuilles possédant à la fois une action parfumante et insecticide. A l'aide de la feuille ainsi obtenue, on peut donc fabriquer des housses destinées à contenir et protéger valablement des vêtements pendant une période prolongée.

## Exemple 5

Parfumage d'un emballage pour cigarettes

On a préparé un emballage pour cigarettes en utilisant une feuille obtenue comme indiqué à l'Exemple 1A. La feuille a été parfumée dans l'une de ses couches (intérieure) à l'aide d'une composition de type chocolat (Chocolat 660.402/B ; origine : Firmenich SA) à raison de 0,4% en poids. A l'ouverture de l'emballage, on a pu observer un dégagement de vapeurs volatiles qui parfumaient agréablement l'atmosphère environnante. Un effet tout à fait similaire a été observé en utilisant une composition de type menthol (Menthe Naefco ; origine : Firmenich SA) à raison de 0,5% en poids.

## Exemple 6

Aromatisation d'un emballage pour snacks extrudés

Un emballage pour snacks de maïs extrudés à goût neutre a été préparé à l'aide d'une feuille obtenue comme indiqué à l'Exemple 1A et aromatisée au moyen d'une composition de type fraise (Fraise 660.478 ; origine : Firmenich SA) à raison de 0,5% en poids. Après quelques jours déjà, les snacks avaient acquis un goût prononcé de fraise. Après 10 mois de stockage dans un emballage scellé, le goût était toujours marqué et inaltéré.

## Exemple 7

Aromatisation d'un emballage pour chocolats, bonbons et biscuits

Des échantillons de chocolat (pralinés), bonbons et biscuits du commerce ont été déballés de leur emballage d'origine et remballés dans des feuilles obtenues selon le procédé décrit à l'Exemple 1A. Ci-après sont indiqués les compositions utilisées pour leur aromatisation et les effets observés lors de la dégustation après 10 mois de stockage dans l'emballage aromatisé.

| 1. Chocolat au lait (Frey Sport Mocca Nuss) | Chocolat 660.403 (0,3%) | goût de chocolat plus prononcé |
|---|---|---|
| 2. Chocolat café-mocca (Frey) | Café 660.447 (0,3%) | odeur de café plus marquée |
| 3. Caramel (Prima Mocca Migros) | Café 660.447 (0,3%) | odeur plus prononcée, goût plus marqué |
| 4. Gaufrettes creuses (Mocca Migros) | Café 660.447 (0,3%) | goût plus intense |
| 5. Dragées au chocolat (Frey) | Chocolat 660.403 (0,3%) | très grande différence d'odeur, goût plus prononcé |

Les effets ont été observés par comparaison avec les échantillons d'origine.
Les compositions aromatisantes utilisées ont pour origine Firmenich SA, Genève.


## Exemple 8


On a procédé à la préparation d'un emballage sous forme de brique, destiné à contenir du jus d'orange à l'aide d'une feuille sandwich obtenue comme décrit à l'Exemple 1B et dont la couche polyoléfinique avait été aromatisée avec une base orange (origine : Firmenich SA; Orange 51.941/A) à raison de 0,4%. Du jus d'orange commercial (qualité Migros-naturel) a été emballé dans la brique obtenue et stocké pendant un mois dans un réfrigérateur, puis comparé avec un échantillon de jus d'orange de qualité identique emballé dans un emballage d'origine constitué par une feuille laminée papier-aluminium-Surlyn.
L'évaluation organoleptique effectuée par 12 experts a montré que la boisson emballée dans la feuille sandwich suivant l'invention possédait une note plus fraîche et plus aromatique.


## Exemple 9


On a procédé à l'emballage d'un jus de pomme du commerce (origine Migros-limpide) de façon identique à celle qui a été indiquée à l'Exemple 8 et la boisson a été stockée pendant un mois dans un réfrigérateur puis comparée avec un échantillon de jus de pomme du commerce. La couche polyoléfinique de la feuille sandwich avait été aromatisée avec un arôme pomme (origine : Firmenich SA ; Pomme 52.681/T) à raison de 0,4%.
Les membres du panel appelés à se prononcer sur les qualités organoleptiques des deux produits ont trouvé que la boisson emballée dans la feuille sandwich suivant l'invention possédait une note plus fraîche et aromatique, ainsi qu'un caractère moins douceâtre que la boisson d'origine.


**Revendications**

1. Feuille sandwich laminée en couches multiples comportant deux couches au moins superposées et accolées par soudure ou collage et dont l'une d'entre elles est constituée par un matériau imperméable aux agents volatils actifs d'une composition parfumante ou désodorisante, d'un arôme ou d'une substance insecticide ou bactéricide et l'autre se compose d'un film réalisé à partir d'un polymère ou d'un copolymère à base de polyoléfine ou d'un copolymère de polyamide et polyéther d'une épaisseur de 10 à 300 microns, lequel film contient, en phase dispersée et homogène, une base constituée par une composition parfumante ou désodorisante, un arôme ou une substance insecticide ou bactéricide dans une proportion de 0,1 à 50% en poids par rapport au poids de la base polymérique dans laquelle elle est incorporée.

2. Feuille sandwich selon la revendication 1, caractérisée en ce que l'une des couches au moins se compose d'un film réalisé à partir de polyéthylène, de polypropylène, de copolymères d'éthylène et propylène, d'éthylène et acétate de vinyle ou d'éthylène et acrylate d'éthyle.

3. Feuille sandwich selon la revendication 1, caractérisée en ce que la couche imperméable aux agents volatils actifs est réalisée à partir d'une résine polyamidique ou polyester, ou d'une feuille d'aluminium.

4. Feuille sandwich selon la revendication 1, caractérisée en ce qu'elle comporte deux couches superposées et accolées par collage à l'aide d'une substance adhésive.

5. Feuille sandwich selon la revendication 4, caractérisée en ce que la substance adhésive est préalablement mélangée à une base constituée par une composition parfumante ou désodorisante, un arôme ou une substance insecticide ou bactéricide.

6. Procédé pour la fabrication d'une feuille sandwich selon la revendication 1, caractérisé en ce qu'on effectue une extrusion séparée et simultanée, dans un courant d'air ou de gaz inerte, d'au moins deux matériaux polymériques dont l'un, à base d'une résine polyoléfinique ou réalisée à partir d'un copolymère de polyamide et polyéther est mélangé préalablement de façon homogène à une base active constituée par une composition parfumante ou désodorisante, un arôme ou une substance insecticide ou bactéricide et l'autre est constitué par un matériau polymérique imperméable aux agents volatils de ladite base active, et en ce que l'extrusion donne lieu à un film polymérique à couches multiples accolées et disposées de sorte que la couche extérieure soit constituée par le matériau polymérique imperméable et la couche intérieure soit constituée par le matériau polymérique contenant la base active.

7. Emballage comportant une feuille sandwich selon la revendication 1.

Fig. 1

Fig. 2

Fig. 5

Fig. 3

Fig. 4

Fig. 6